# EUROPEAN PATENT SPECIFICATION

(11) **EP 4 637 620 B1**
(45) Date of publication and mention of the grant of the patent: **26.08.2026**
(21) Application number: 23836858.3
(22) Date of filing: 21.12.2023
(51) Int. Cl.: A61C 5/64, A61C 5/62, A61C 5/60, A61B 17/88, A61B 17/00

(54) **BONE ADHESIVE APPLICATOR**
APPLIKATOR FÜR KNOCHENKLEBER
APPLICATEUR D'ADHÉSIF POUR OS

(30) Priority: 21.12.2022 EP 22215548
(43) Date of publication of application: 29.10.2025
(60) Divisional application: 26192680.2
(73) Proprietor: Institut Straumann AG, 4002 Basel (CH)
(72) Inventor: ZOFFMANN ANDERSEN, Ole, 4002 Basel (CH); BELLON PECECNIK, Benjamin, 4002 Basel (CH); RENGGLI, Birgit, 4002 Basel (CH); KEANE, Robert, 4002 Basel (CH)
(74) Representative: Schaad, Balass, Menzl & Partner AG
(86) International application number: PCT/EP2023/087205
(87) International publication number: WO 2024/133638

(56) References cited:
- US-A1- 2008 203 112
- US-B1- 6 386 872

## Description

### Field of the invention

The present invention relates to a bone adhesive applicator for mixing a bone adhesive powder with a liquid to obtain a bone adhesive and applying the bone adhesive, and a method for preparing a bone adhesive.

### Background of the invention

The use of a bone adhesive to glue implants in place may be considered as a favorable advancement in the field of implantology. Together with the ability to robustly bond to mineralized bone tissue and/or to metal, a suitable bone adhesive can act as an initial implant stabilization glue with reduced detrimental effect on the bone while placing of the implant.

Typically, a bone adhesive comprises at least one dry component which is mixed with a liquid prior to application. Since after mixing of the dry component with the liquid, the bone adhesive hardens within a rather short time scale on the order of a few minutes, the dry component has to be kept separate from the liquid until mixing can be performed shortly before application of the bone adhesive.

A package comprising a device for storing, mixing, and administering an adhesive composition is for example described in WO 2021/041943 A1. The device comprises a chamber within a main body, a barrier, a plunger, a nozzle, and an adhesive composition, wherein the package limits ingress of a contaminating element. The device may comprise a first chamber and a second chamber separated by the barrier, wherein components of the composition may be provided as dry components, such as powder or granules within the first chamber and the second chamber may comprise an aqueous medium, such as water. Specifically, pressure is applied to the plunger of the device to breach the barrier, allowing the components of the adhesive composition to come into contact with each other. The device is then placed into an agitator to agitate the contents of the device, thereby preparing the adhesive composition. The chamber in which the components of the composition are mixed can be swept by a plunger that empties the chamber by expressing the chamber contents out through an orifice of the main body to an application site, such as bone, or into a nozzle through which the resulting implantable composition flows to an application site, such as bone.

US2008203112 is directed to a system and method which enable airfree filling and dispensing of substances using a compartment with a displaceable piston, a movable front plug, and a vent to seal volumes.

### Summary of the invention

When using a bone adhesive to glue an implant at an application site, it is desired to provide the bone adhesive powder separate from the liquid before mixing. It is further desired that bringing into contact of the separately stored bone adhesive powder and liquid is enabled in an effortless fashion, in order to provide ease of use for a device used for mixing and applying the bone adhesive to glue the implant. For implants in the oral cavity, i.e. dental implants, respectively, mixing and applying the bone adhesive can particularly challenging due to the small amounts of bone adhesive involved therewith.

It is therefore an object of the invention to provide a bone adhesive applicator for mixing a bone adhesive powder with a liquid to obtain a bone adhesive and applying the bone adhesive, and a method for preparing a bone adhesive, which at least partially improve the prior art and avoid at least part of the disadvantages of the prior art.

According to the present invention, this object is achieved by the features of the independent claims. In addition, further advantageous embodiments follow from the dependent claims and the description as well as the figures.

According to an aspect of the invention, this object is particularly achieved by a bone adhesive applicator for mixing a bone adhesive powder with a liquid to obtain a bone adhesive and applying the bone adhesive, comprising a housing with a longitudinal axis and an outlet, a mixing compartment arranged in the housing and in fluidic communication with the outlet, an axially movable plunger arranged in the housing and axially delimiting the mixing compartment opposite to the outlet, wherein the mixing compartment comprises the bone adhesive powder and is fluidically connectable to a reservoir comprising the liquid, wherein the plunger is configured to fluidically separate in a first position the reservoir and the mixing compartment and to fluidically connect in a second position the reservoir and the mixing compartment.

Besides the function of conveying the bone adhesive out of the mixing compartment by advancing the plunger in the housing towards the outlet, the plunger can thus provide the function of a valve element configured to selectively fluidically separate and connect the reservoir and the mixing compartment. Therefore, a multi-functional plunger can be provided, which allows to reduce the structural complexity of the bone adhesive applicator since a separate element for fluidically separating and connecting the reservoir and the mixing compartment, such as for example a tearable barrier or membrane, may not be required. By reducing the complexity of the structure of the bone adhesive applicator, reliability of the bone adhesive applicator can be increased due to the reduced number of parts that may fail or be prone to damage. Further, ease of use can be increased, as a user of the bone adhesive applicator does not have to operate a separate element for connecting the reservoir and the mixing compartment but can use the plunger for this purpose.

This may particularly be advantageous for implants in the oral cavity where typically only small amounts of bone adhesives are used. In some embodiments, the bone adhesive applicator according to the present disclosure is therefore a dental bone adhesive applicator for mixing a bone adhesive powder with a liquid to obtain a dental bone adhesive and applying the dental bone adhesive. Reducing the structural complexity of the bone adhesive applicator while providing accuracy in applying the bone adhesive may be advantageous for small amounts of bone adhesives. Further, the reservoir and/or the mixing compartment can be adapted for the use with small amounts of bone adhesive used for dental implants. Increasing ease of use, as mentioned above, may also be advantageous for a user such as a dental surgeon applying the dental bone adhesive using the dental bone adhesive applicator. As described further below, the dental bone adhesive applicator provides particular advantage for a dental bone adhesive where the volume of the liquid is smaller than the volume of the bone adhesive powder.

In the context of the present disclosure, the term "bone adhesive" shall be understood to comprise not only an adhesive such as a bone glue, but also or alternatively a bone cement. In particular, the bone adhesive may be a composition which is configured to create a bond between bone and bone and/or bone and implant.

In some embodiments, the bone adhesive applicator comprises a mixer configured to mix the bone adhesive powder and the liquid in the mixing compartment, wherein the mixer comprises a mixing head arranged in the mixing compartment and a connector connected to the mixing head and comprising a drive interface.

The mixing head may be driven by a drive which can be connected to the drive interface of the connector. The drive may be used to rotate and/or vibrate the mixing head such that an efficient mixing of the components of the bone adhesive can be provided.

Arranging the mixing head in the mixing compartment provides the advantage that the bone adhesive applicator which performs mixing and applying of the bone adhesive can be designed in a compact fashion, as compared to for example devices where mixing of the bone adhesive is performed by a separate agitator into which a barrel with the mixture is clamped for preparing the bone adhesive.

The bone adhesive may be a dual part composition comprising an aqueous solution as first component and a self-setting adhesive powder as second component, wherein said self-setting adhesive powder comprises at least a multivalent metal salt and phosphoserine. The multivalent metal salt may comprise calcium phosphate, for example tetra calcium phosphate (TTCP) or tri calcium phosphate (TCP), preferably α-TCP. The self-setting adhesive powder may comprise 50-80% by weight of TCP or TTCP, phosphoserine and preferably further components like calcium silicate. Preferably, the self-setting adhesive powder comprises 65 to 80 % by weight of α-TCP, 20-30% by weight of phosphoserine and 1 to 5 % by weight of calcium silicate. The dental bone adhesive may be formed by mixing the first component and the second component and allowing the obtained composition sufficient time to set. Initial setting may typically be achieved within 15 seconds and 90 seconds, and final setting may typically be achieved within 1 to 15 minutes.

Preferably, a smaller amount, especially volume, of liquid may be used for the bone adhesive than the amount, especially volume, of bone adhesive powder. In some embodiments, the volume ratio of the liquid used for the bone adhesive to the bone adhesive powder used may be between 1:3 and 1:6.

Especially for the use as a dental bone adhesive applicator, the reservoir may advantageously be designed for the small amount of liquid involved for preparing the dental bone adhesive.

The mixing compartment being axially delimited by an axially movable plunger provides the advantage that the volume of the mixing compartment may be varied. Besides advancing the plunger to convey the dental bone adhesive out of the mixing compartment, the plunger may also be moved towards the outlet in order to reduce the volume of the mixing compartment. The dental bone adhesive applicator may therefore accommodate a volume decrease of the mixture of the components of the dental bone adhesive, which may for example occur upon contact or mixing of the different components of the dental bone adhesive, by appropriate movement of the plunger.

In some embodiments, the mixing head is a brush, preferably comprising bristles made of a biodegradable material.

By using a brush with flexible bristles, a radial collapsibility of the mixing head can be achieved. In particular, the brush may comprise a shaft with bristles mounted thereon such that the bristles may be bended against the shaft, thereby enabling the mixing head to radially collapse. Resilient bristles may further enable the mixing head to re-expand after being collapsed.

By using a radially collapsible mixing head, such as a brush, introducing and removing the mixing head through the outlet, which has a smaller cross-section than the housing, can be enabled while providing efficient mixing within the mixing compartment by re-expanding the mixing head after passage through the outlet.

In particular, the mixing head may provide efficient mixing of a bone adhesive where the volume of the liquid is smaller than the volume of the bone adhesive powder.

Bristles made of a biodegradable material provide the advantage that bristles which may detach from the brush while mixing may biodegrade when being applied together with the bone adhesive. In some embodiments, the bristles may be made of chitosan.

In some embodiments, the connector is guided through the outlet such that the drive interface is arranged outside of the housing, wherein the drive interface is configured for connection with a dental handpiece.

After mixing the dental bone adhesive, the mixing head and part of the connector may be removed from the mixing compartment, such that the dental bone adhesive may be applied through the outlet by advancing the plunger towards the outlet.

With the drive interface being connectable to a dental handpiece such that the mixer can be driven by a drive of the dental handpiece, operation of the dental bone adhesive applicator can substantially be facilitated, as no separate drive may be required and an existing dental handpiece can be used to drive the mixer.

In some embodiments, the bone adhesive applicator comprises a cap releasably mounted on the outlet, wherein the cap comprises a feedthrough for the connector.

The cap may be connected with the outlet by a thread or a snapfit connection. After mixing, the cap and the mixer may be removed from the housing such that the bone adhesive may be conveyed through the outlet out of the mixing compartment.

In some embodiments, the housing comprises a lateral inlet configured for fluidic communication of the mixing compartment with the reservoir, wherein the fluidic communication through the lateral inlet is interruptible by the plunger in the first position of the plunger.

A lateral inlet provides the advantage that a lateral face or a portion of the lateral face of the plunger can be used to selectively separate or connect the reservoir and the mixing compartment. Thus, multifunctionality of the plunger can be achieved by using the front face of the plunger for conveying the bone adhesive out of the mixing compartment by advancing of the plunger and by using a lateral face of the plunger for the function of a valve element between the reservoir and the mixing compartment.

In some embodiments, the lateral inlet comprises an aperture in a lateral wall of the housing, wherein the aperture is fluidically closable by the plunger in the first position of the plunger.

The aperture may be used to fluidically connect a reservoir comprising a liquid with the housing. The liquid can be kept separate from the bone adhesive powder by closing the aperture with the plunger in a first position. By moving the plunger into a second position, the aperture can be opened and the liquid brought into contact with the bone adhesive powder.

The aperture preferably exhibits a diameter which is smaller than or equal to an axial diameter of the plunger.

Complete closing of the aperture can therefore be achieved by simply moving the plunger into an adequate first position where the lateral face of the plunger completely closes the aperture.

In some embodiments, the bone adhesive applicator comprises the reservoir which is arranged at an outer face of the housing.

In particular, the reservoir may extend at least partially along the lateral outer face of the housing, especially along the longitudinal axis of the housing.

Thus, an integrated bone adhesive applicator including a reservoir can be provided without requiring the user to keep a separate reservoir which first has to be mounted and/or connected to the housing before bringing the liquid into contact with the bone adhesive powder.In some embodiments, the reservoir is a bag or a container.

Preferably, the diameter of the bag or container perpendicular to the wall of the housing is smaller than the transverse diameter of the housing of the bone adhesive applicator. Preferably, the diameter of the bag or container perpendicular to the wall of the housing is smaller than half or one third or one quarter of the transverse diameter of the housing of the bone adhesive applicator. The reservoir, and in particular the bag or container, can therefore be designed in a rather flat fashion which improves compactness of the bone adhesive applicator. By extending the reservoir along the lateral outer face of the housing, however, a sufficient volume of the liquid can be enabled while providing a flat reservoir.

As described above, this may particularly be advantageous for a dental bone adhesive applicator where a small volume of liquid is used, especially where the volume of the liquid is smaller than the volume of the bone adhesive powder.

In some embodiments, the volume of the reservoir is smaller than the volume of the mixing compartment for the plunger being in the first position.

The ratio of the volume of the reservoir to the volume of the mixing compartment for the plunger being in the first position may be between 1:3 and 1:6.

In some embodiments, the volume of the reservoir is between 100 µl and 150 µl.

In some embodiments, the container comprises a longitudinal axis and an axially movable plunger. The container may therefore be designed as a syringe.

Preferably, the reservoir comprises a reservoir outlet which is flush with the aperture of the lateral inlet.

By designing the reservoir outlet to be flush with the aperture of the lateral inlet, transfer of the liquid from the reservoir to the mixing compartment can be improved and leakage may be prevented or minimized.

The mixing compartment and the plunger may be configured to generate an intake pressure for liquid at the lateral inlet by moving the plunger away from the outlet.

Liquid from the reservoir can therefore be drawn into the mixing compartment by retracting the plunger from a first position into a second position where the lateral inlet is at least partially arranged between the plunger and the outlet with respect to the longitudinal axis of the housing. Preferably, the housing is dimensioned that the plunger can be retracted until the reservoir is emptied and all liquid has been transferred to the mixing compartment.

As the liquid can be drawn from the reservoir into the mixing compartment by retracting the plunger, additional reservoir elements such as a reservoir plunger, a pump or similar elements to transfer the liquid from the reservoir to the mixing compartment can be omitted.

In some embodiments, the reservoir comprises a pressure compensating valve in order to compensate the intake pressure at the lateral inlet drawing the liquid out of the reservoir.

In some embodiments, a check valve is arranged at the lateral inlet or the reservoir outlet, wherein the check valve is configured to provide substantially only flow into the housing or to provide a larger flow into the housing than out of the housing.

By providing a check valve, reentering of liquid that has been transferred to the mixing compartment into the reservoir while mixing of the liquid with the bone adhesive powder or advancing the plunger towards the outlet can be reduced or avoided. Further, entering of bone adhesive into the reservoir when pushing the plunger towards the outlet in order to convey the bone adhesive out of the mixing compartment, can be reduced or avoided.

In some embodiments, the check valve comprises a flap with an edge portion mounted at a side of the aperture proximal to the plunger in the second position.

The flap can therefore be opened when the plunger is retracted from a first position into a second position such that liquid from the reservoir can be drawn into the mixing compartment. When advancing the plunger again from the second position towards the outlet, the flap is moved towards the aperture such that entering of liquid and/or bone adhesive into the reservoir can be reduced or minimized.

In some embodiments, a cross-sectional area of the aperture of the lateral inlet is smaller than a cross-sectional area of the outlet, preferably with a ratio of smaller than 1:5, particularly preferably smaller than 1:10.

By designing the cross-sectional area of the aperture of the lateral inlet to be smaller than the cross-sectional area of the outlet, the flow of bone adhesive out of the outlet can be maintained to be larger than a reflow or leakage, respectively, of bone adhesive into the reservoir.

In some embodiments, the lateral inlet comprises an interface configured for connection with a reservoir interface.

The reservoir may therefore be manufactured separately and filled with the liquid before connecting to the housing. For certain applications, the reservoir may be provided separately and the user may connect the reservoir before transferring of the liquid to the mixing compartment. For certain applications, the user may exchange the reservoir with another reservoir containing a different liquid.

In some embodiments, the interface comprises a thread or one or more catches and/or recesses.

In some embodiments, the lateral inlet comprises a bulge of a lateral wall of the housing such that an inner diameter of the housing is increased at the bulge.

By providing a bulge of the lateral wall of the housing, a fluid bypass for the mixing compartment around the plunger can be formed, as the housing exhibits at the bulge an inner cross-sectional diameter which is larger than the transverse diameter of the plunger. By advancing the plunger to the position of the bulge, the mixing compartment can therefore fluidically be connected to a reservoir through the fluid bypass formed by the bulge. The fluidic connection can be interrupted by the plunger being at least partially positioned in a bulge-free portion of the housing, thereby closing the fluid bypass.

In some embodiments, the bulge extends only around a part of the circumference of the housing, preferably around less than one sixteenth, one eighth or one quarter of the circumference of the housing.

By extending the bulge only around a part of the circumference of the housing, stable radial positioning of the plunger within the housing can be ensured while providing a fluid bypass around the plunger.

In some embodiments, the bone adhesive applicator comprises two, three, four or more bulges which are evenly arranged around the circumference of the housing, wherein the bulges preferably each extend around less than one sixteenth, one eighth or one quarter of the circumference.

By providing two, three, four or more bulges, the flow of the liquid from the reservoir to the mixing compartment can be improved. By arranging the two, three, four or more bulges evenly around the circumference of the housing, the flow of the liquid into the mixing compartment can be more evenly distributed around the circumference of the housing, which is beneficial for the mixing of the liquid and the bone adhesive powder.

In some embodiments, the bone adhesive applicator comprises an axially movable auxiliary plunger arranged in series with the plunger such that the plunger is arranged between the auxiliary plunger and the outlet.

The bone adhesive applicator may comprise a plunger rod connected to the auxiliary plunger such that the auxiliary plunger can be advanced or retracted by moving the plunger rod.

In some embodiments, the reservoir is formed between the auxiliary plunger and the plunger.

The reservoir can therefore be formed within the housing. By forming the reservoir to be contained within the housing, compactness of the bone adhesive applicator can be improved. Further, structural complexity can be reduced, as a separate bag or container for the reservoir is not required and the lateral walls of the reservoir are formed by the housing.

In the first position, the plunger may be at least partially arranged in a portion of the housing adjacent to an axially distal end of the bulge with respect to the outlet.

Thus, the fluid bypass formed by the bulge can be closed by the plunger in the first position and connection to the reservoir be interrupted due to the plunger at least partially being arranged in a bulge-free portion of the housing.

The auxiliary plunger may be configured to displace the plunger into the second position by moving towards the plunger.

By moving towards the plunger, the auxiliary plunger can apply a pressure onto the liquid of the reservoir which in turn effects a pressure onto the plunger such that the plunger may be advanced into a second position. The plunger can therefore hydraulically be displaced by moving the auxiliary plunger due the reservoir being formed between the auxiliary plunger and the plunger.

The plunger can therefore function as a hydraulic valve element to close and open the fluid bypass, wherein the plunger can hydraulically be actuated by the auxiliary plunger using the liquid contained in the reservoir formed between the auxiliary plunger and the plunger. The arrangement of the bulge, plunger and auxiliary plunger provides the advantage that a separate liquid for hydraulic actuation of the plunger is not required, as the liquid which is to be transferred into the mixing compartment can be used for transmitting the pressure generated by the auxiliary plunger and for actuating of the plunger.

In the second position, the plunger may be arranged at the bulge such that a fluid bypass is formed between the reservoir and the mixing compartment, wherein the auxiliary plunger is configured to transfer liquid from the reservoir to the mixing compartment through the bypass by moving towards the plunger.

The fluid bypass can therefore be opened by the plunger in the second position such that the reservoir and the mixing compartment are connected by the fluid bypass. After opening the fluid bypass, the pressure generated by the advancing auxiliary plunger can be used to convey the liquid out of the reservoir through the fluid bypass into the mixing compartment. While transferring the liquid out of the reservoir, the plunger may continue to advance towards the outlet due to part of the pressure still being applied onto the plunger by the advancing auxiliary plunger.

The auxiliary plunger can therefore be used for displacing the plunger blocking the fluid bypass in order to open the fluid bypass and to transfer the liquid from the reservoir into the mixing compartment once the fluid bypass is opened.

In some embodiments, the auxiliary plunger is movable towards the plunger until contacting the plunger.

By moving the auxiliary plunger towards the plunger until contacting the plunger, the reservoir can be emptied, and the entire liquid be transferred to the mixing compartment. By continuing to advance the auxiliary plunger after contacting the plunger, the plunger can concomitantly be advanced and thereby convey the bone adhesive through the outlet out of the mixing compartment.

The bulge may have an axial length which is equal or larger than the stroke of the plunger between opening of the fluid bypass and contacting of the auxiliary plunger and the plunger.

Due to the axial length of the bulge being equal or larger than the stroke of the plunger between the position of the plunger when opening of the fluid bypass and the position of the plunger when the auxiliary plunger contacts the plunger, it can be ensured that the fluid bypass is open until the reservoir is emptied or largely emptied.

The one or more bulges are therefore preferably dimensioned that the auxiliary plunger contacts the plunger before the plunger reaches an axially proximal end of the bulge with respect to the outlet.

Accordingly, the one or more bulges may be dimensioned to provide a sufficiently large cross section for the liquid flow such that the auxiliary plunger may empty the reservoir before the plunger reaches the axially proximal end of the bulge and closes the fluid bypass.

The auxiliary plunger may have an axial diameter which is larger than the axial length of the bulge.

By using an auxiliary plunger with a larger axial diameter than the axial length of the bulge, backflow of liquid through the fluid bypass can be avoided when the front face of the auxiliary plunger with respect to the outlet is positioned at the bulge.

According to a further aspect, the present invention is also directed to a method for preparing a bone adhesive, comprising the steps of: providing a bone adhesive applicator according to the present disclosure; supplying the liquid from the reservoir to the mixing compartment by moving the plunger from the first position to the second position; connecting a drive to the drive interface of the connector; mixing the liquid with the bone adhesive powder in the mixing compartment by actuating the mixer using the drive.

After mixing of the liquid with the bone adhesive powder, the mixer may be removed from the bone adhesive applicator and the bone adhesive be applied by advancing the plunger towards the outlet such that the bone adhesive is conveyed out of the mixing compartment.

In some embodiments of the method, the drive is a drive of a dental handpiece.

### Brief description of the drawings

The present invention will be explained in more detail, by way of exemplary embodiments, with reference to the schematic drawings, in which:
- Fig.1: shows a side view of an embodiment of a bone adhesive applicator with the plunger in a first position;
- Fig.2: shows a top view of the bone adhesive applicator of Fig.1;
- Fig.3: shows a side view of the bone adhesive applicator of Fig.1 with the plunger in a second position;
- Fig.4: shows the bone adhesive applicator of Fig.3 after mixing the liquid and the bone adhesive powder;
- Fig.5: shows a side view of an embodiment of a bone adhesive applicator with a check valve at the lateral inlet;
- Fig.6: shows a side view of an embodiment of a bone adhesive applicator with the housing comprising a bulge and the plunger in a first position;
- Fig.7: shows the bone adhesive applicator of Fig.6 with the plunger being advanced into a second position;
- Fig.8: shows the bone adhesive applicator of Fig.7 after mixing the liquid and the bone adhesive powder.

### Detailed description of exemplary embodiments

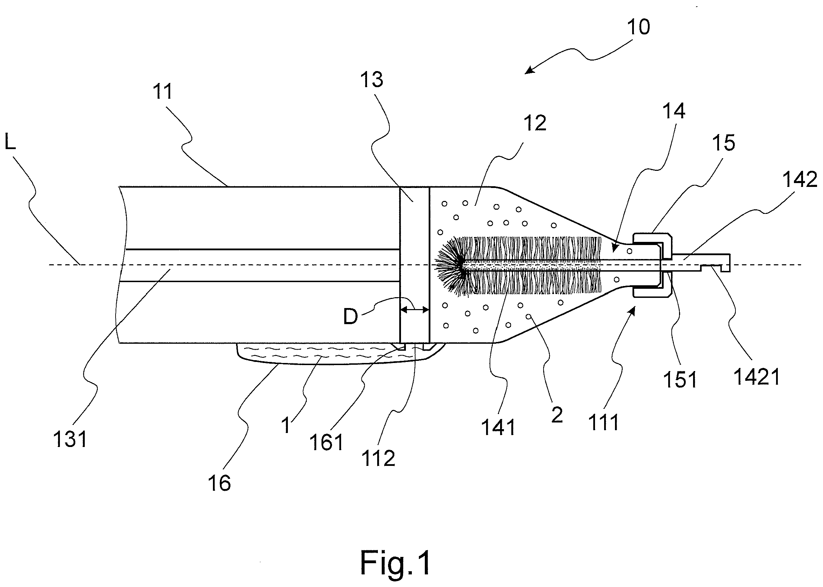

Figure 1 shows a side view of an embodiment of a bone adhesive applicator in an example of a dental bone adhesive applicator 10. The dental bone adhesive applicator 10 comprises a housing 11 with a longitudinal axis L and an outlet 111. An axially movable plunger 13 is arranged in the housing 11. The plunger 13 is movable by a plunger rod 131 connected to the plunger 13. The dental bone adhesive applicator 10 comprises a mixing compartment 12 which is axially delimited by the plunger 13 opposite to the outlet 111. A bone adhesive powder 2 is contained in the mixing compartment 12.

The dental bone adhesive applicator 10 further comprises a mixer 14 with a mixing head 141 in the form of a brush arranged in the mixing compartment 12. The bristles of the brush 141 are biodegradable. The mixer 14 further comprises a connector 142 comprising a drive interface 1421 which is arranged outside of the housing 11 as the connector 142 is guided through the outlet 111. The drive interface 1421 is configured for connection with a dental handpiece (not shown in Figure 1).

A cap 15 with a feedthrough 151 closes the outlet 111. The feedthrough 151 is dimensioned to receive the connector 142 therethrough. The cap 15 is mounted onto the outlet 111 by a thread.

Due to the flexible bristles of the brush, the mixing head 141 is radially collapsible and can be removed from the mixing compartment 12 after mixing the dental bone adhesive by retracting the mixer 14 through the outlet 111.

The dental bone adhesive applicator 10 further comprises a reservoir 16 which is arranged at a lateral outer face of the housing 11 and contains a liquid 1. The reservoir 16 is in the form of a bag and comprises a reservoir outlet 161 which is flush with a lateral inlet 112. The lateral inlet 112 is formed by an aperture in a lateral wall of the housing 11. In Figure 1, the plunger 13 is in the first position closing the aperture 112 such that fluidic communication between the reservoir 16 and the mixing compartment 12 is interrupted by the plunger 13. The aperture 112 exhibits a diameter which is smaller than the axial diameter D of the plunger 13 such that the aperture 112 is completely closed by the plunger 13 in the first position.

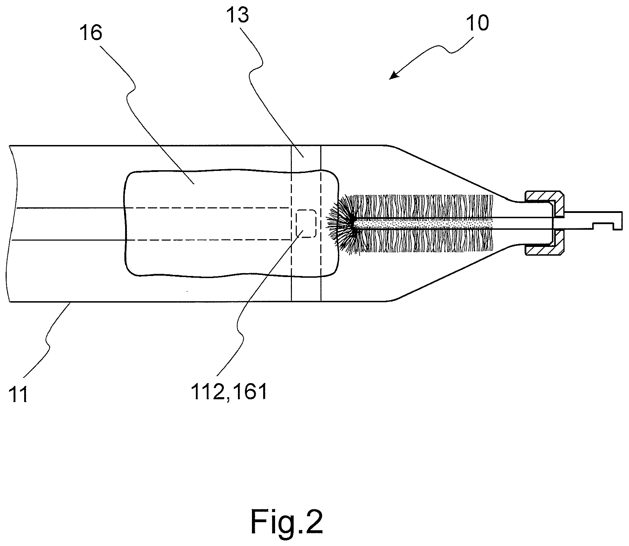

Figure 2 shows a top view of the dental bone adhesive applicator 10 of Figure 1 where the aperture 112 in the housing 11 can be recognized. The plunger 13 is in the first position closing the aperture 112 with its lateral face.

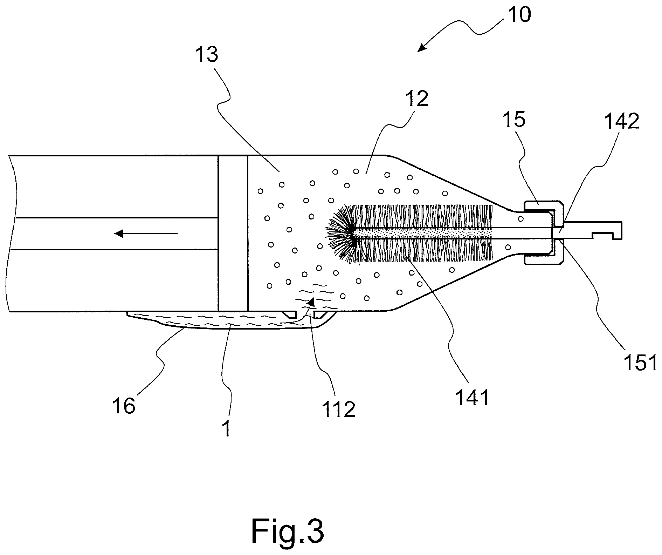

Figure 3 shows a side view of the dental bone adhesive applicator 10 of Figure 1 where the plunger 13 has been moved into a second position, as symbolized by the horizontal arrow. The aperture 112 is thereby opened such that the reservoir 16 is fluidically connected with the mixing compartment 12 and liquid 1 is drawn into the mixing compartment 12, as symbolized by the curved arrow. The volume of the reservoir 16 formed as a bag is decreased as compared to Figure 1 due to the flexibility of the bag forming the reservoir 16 and accommodating the intake pressure at the lateral inlet 112. By continuing the retracting motion of the plunger 13, the intake pressure can be maintained such that the liquid 1 of the reservoir 16 is completely drawn into the mixing compartment 12.

The feedthrough 151 of the cap 15 is dimensioned to receive the connector 142 in a sufficiently close fit to allow the intake pressure to be built up at the lateral inlet 112. In some embodiments, a sealing element may be arranged at the feedthrough 151. However, the feedthrough 151 is dimensioned such that the mixing head 141 can rotate around the longitudinal axis L. In some embodiments, the connector 142 may comprise a hollow shaft within which a rotatable rod connected with the mixing head is received.

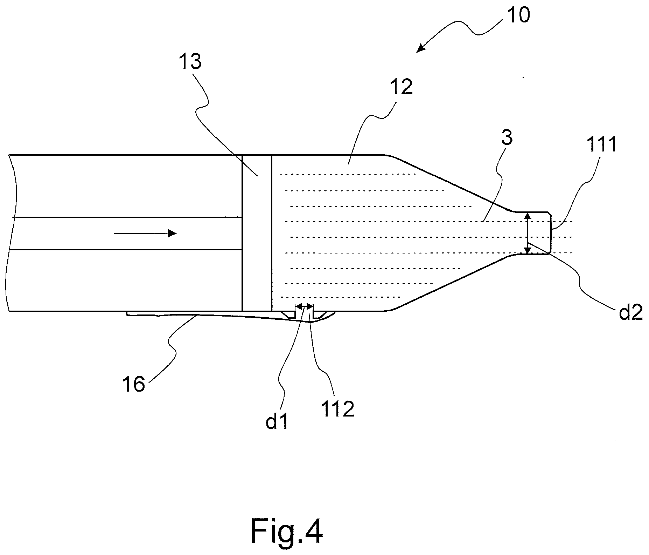

Figure 4 shows the dental bone adhesive applicator 10 of Figure 3 after mixing the liquid and the bone adhesive powder such that the dental bone adhesive 3 is obtained in the mixing compartment 12. The cap has been removed together with the mixer such that the outlet 111 is open for conveying the dental bone adhesive 3 out of the mixing compartment 12. The reservoir 16 is collapsed as the liquid has been transferred to the mixing compartment 12. The dental bone adhesive 3 is conveyed out of the mixing compartment 12 by advancing the plunger 13 towards the outlet 111, as symbolized by the horizontal arrow.

The diameter d1 and thus the cross-sectional area of the aperture of the lateral inlet 112 is smaller than the diameter d2 and accordingly the cross-sectional area of the outlet 111. Thus, the flow out of the dental bone adhesive 3 out of the outlet 111 substantially larger than potential leakage of dental bone adhesive 3 back to the reservoir 16.

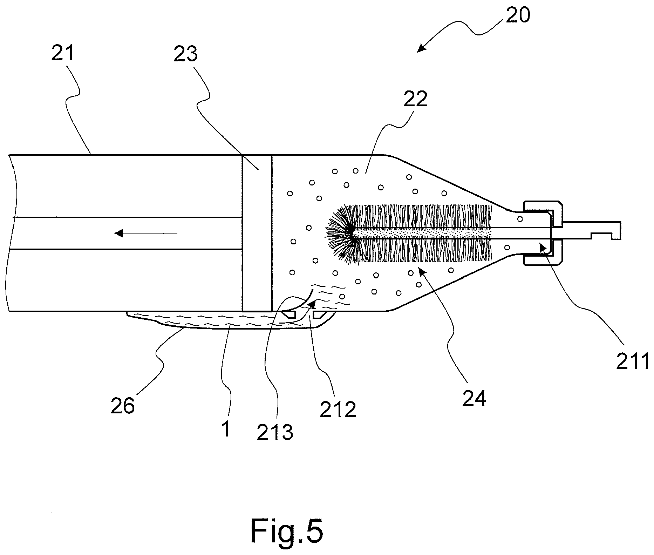

Figure 5 shows a side view of an embodiment of a dental bone adhesive applicator 20 with a flap 213 mounted at a side of the aperture 212 proximal to the plunger 23 being in the second position. When retracting the plunger 23, as symbolized by the horizontal arrow, the flap 213 is opened such that the liquid 1 can be drawn from the reservoir 26 to the mixing compartment 22. By advancing the plunger 23 again from the second position towards the outlet 211 (after mixing the dental bone adhesive and removing of the mixer 24), the flap 213 is moved towards the aperture such that entering of dental bone adhesive into the reservoir can be reduced or avoided. The flap 213 therefore functions as a check valve configured to provide a larger flow into the mixing compartment 22 than into the reservoir 26.

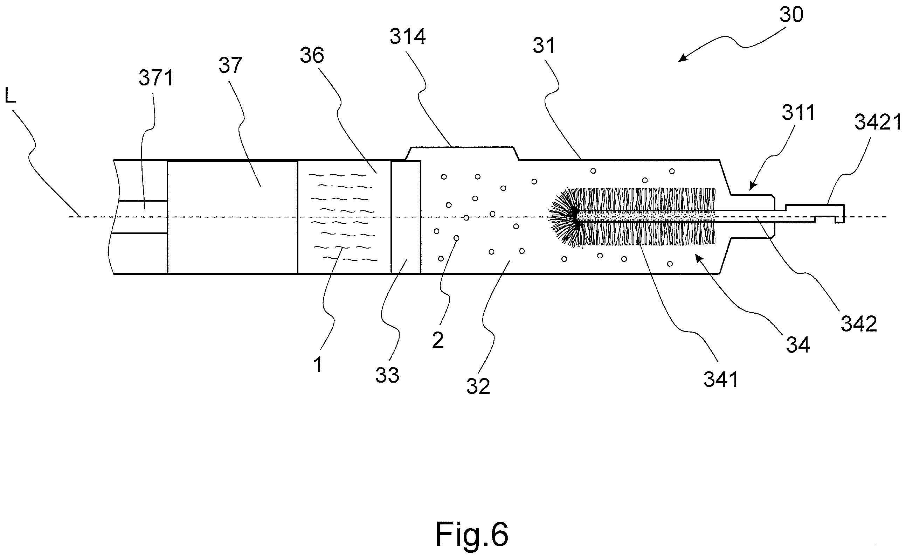

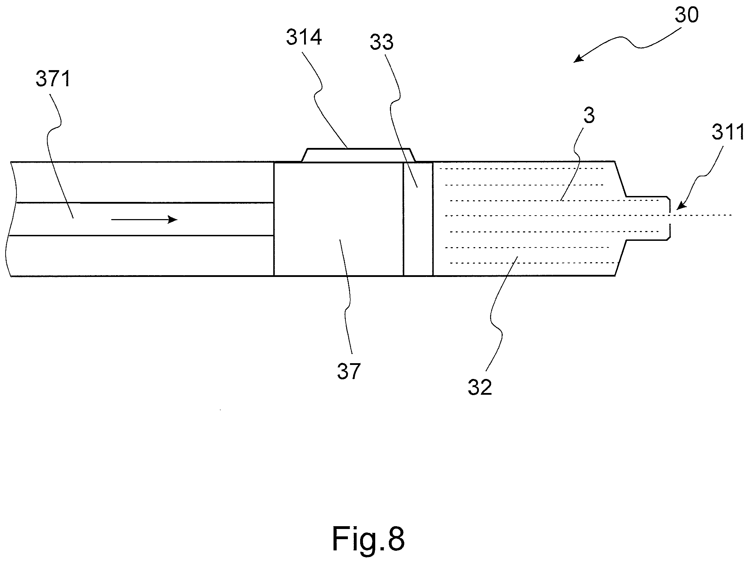

Figure 6 shows a side view of an embodiment of a dental bone adhesive applicator 30 with the housing 31 comprising a bulge 314 where the inner transverse diameter of the housing 31 is increased. The bulge 314 extends only around a part of the circumference of the housing 31, for example less than one eighth of the circumference of the housing 31. The dental bone adhesive applicator 30 comprises a plunger 33 and an auxiliary plunger 37 arranged in series with the plunger 33 such that a reservoir 36 containing a liquid 1 is formed between the auxiliary plunger 37 and the plunger 33. The auxiliary plunger 37 is connected to a plunger rod 371 by which the auxiliary plunger 37 can be advanced and retracted. By advancing the auxiliary plunger 37 towards the plunger 33, the plunger 33 can be advanced due to the liquid 1 contained in the reservoir 36. The axial diameter of the auxiliary plunger 37 is larger than the axial diameter of the plunger 33 and, in particular, larger than the axial length of the bulge 314, which is particularly easily recognizable in Figure 8.

The bulge 314 forms a lateral inlet configured for fluidic communication of the mixing compartment 32 with the reservoir 36. In Figure 6, the lateral inlet formed by the bulge 314 is closed due to the plunger 33 being positioned in a first position where the plunger 33 is partially arranged in the portion of the housing 31 adjacent to the axially distal end of the bulge 314 with respect to the outlet 311. Thus, the fluidic communication between the reservoir 36 and the mixing compartment 32 is interrupted by the plunger 33.

Similar to the embodiments as shown in Figures 1-5, The dental bone adhesive applicator 30 comprises a mixer 34 with a mixing head 341 comprising a brush. The connector 342 of the mixer 34 is guided through the outlet 311 such that the drive interface 3421 of the connector 342 is arranged outside of the housing 31. The outlet 311 is dimensioned such that the connector 342 is received in the outlet 311 by a sufficiently close fit in order to minimize or avoid leakage through the outlet while mixing. In some embodiments, a sealing element may be arranged at the outlet 311. The outlet 311 is dimensioned such that the mixing head 341 can rotate around the longitudinal axis L. In some embodiments, the connector 342 may comprise a hollow shaft within which a rotatable rod connected with the mixing head is received.

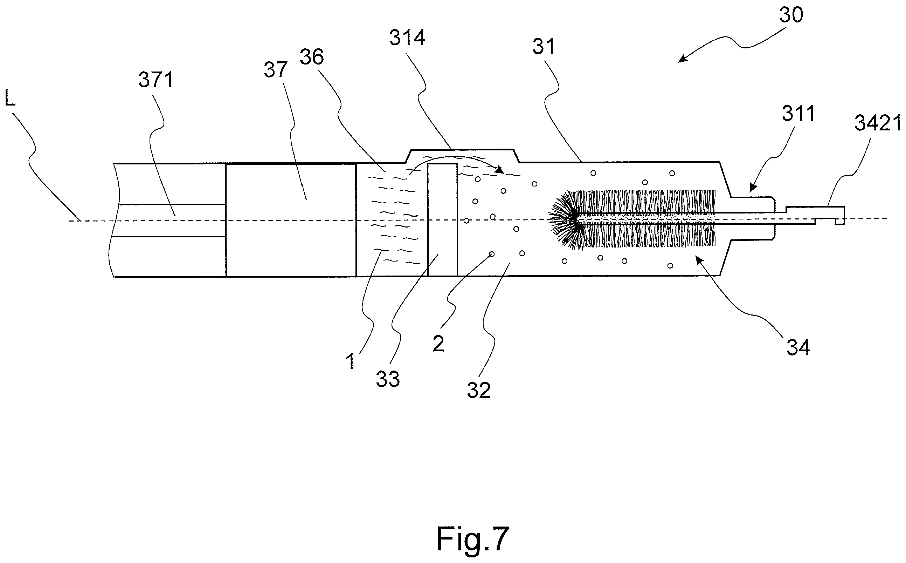

Figure 7 shows the dental bone adhesive applicator 30 of Figure 6 where the auxiliary plunger 37 has been advanced towards the plunger 33 by pushing the plunger rod 371 towards the outlet 311. By advancing of the auxiliary plunger 37, the plunger 33 has also been advanced into a second position at the bulge 314 such that a fluid bypass through the lateral inlet formed by the bulge 314 is opened between the reservoir 36 and the mixing compartment 32, as symbolized by the curved arrow. Thus, the liquid 1 is transferred from the reservoir 36 to the mixing compartment 32 through the bypass by the auxiliary plunger 37 moving towards the plunger 33.

After the liquid 1 is transferred to the mixing compartment 32, the mixer 34 can be driven by a drive connected to the drive interface 3421, for example a drive of a dental handpiece, effecting mixing of the liquid 1 and the bone adhesive powder 2 to obtain the dental bone adhesive.

Figure 8 shows the dental bone adhesive applicator 30 of Figure 7 after mixing the liquid and the bone adhesive powder such that the dental bone adhesive 3 is obtained in the mixing compartment 32. The mixer has been removed from the mixing compartment 32 such that the outlet 311 is open for conveying the dental bone adhesive 3 out of the mixing compartment 32.

The auxiliary plunger 37 has been advanced until contacting the plunger 33. Due to the contacting of the auxiliary plunger 37 and the plunger 33, the reservoir is collapsed as the liquid has completely been transferred to the mixing compartment 12. By moving the plunger rod 371, the auxiliary plunger 37 can be advanced together with the plunger 33 towards the outlet 311 such that the dental bone adhesive 3 is conveyed out of the mixing compartment 32, as symbolized by the horizontal arrow. As mentioned above in connection with Figure 6, the axial diameter of the auxiliary plunger 37 is larger than the axial length of the bulge 314 such that reflow of bone adhesive or liquid through the bulge 314 can be avoided. As also recognizable in connection with Figures 6-8, the axial length of the bulge 314 is equal or larger than the stroke of the plunger 33 between the position of the plunger 33 when opening of the fluid bypass formed by the bulge 314 and the position of the plunger 33 when the auxiliary plunger 37 contacts the plunger 33. An open fluid bypass through the bulge 314 can therefore be maintained until the auxiliary plunger 37 contacts the plunger 33 and all liquid is transferred from the reservoir 36 to the mixing compartment 32.

## Claims

1. A bone adhesive applicator (10, 20, 30) for mixing a bone adhesive powder (2) with a liquid (1) to obtain a bone adhesive (3) and applying the bone adhesive, comprising a housing (11, 21, 31) with a longitudinal axis (L) and an outlet (111, 211, 311), a mixing compartment (12, 22, 32) arranged in the housing and in fluidic communication with the outlet, an axially movable plunger (13, 23, 33) arranged in the housing and axially delimiting the mixing compartment opposite to the outlet, wherein the mixing compartment comprises the bone adhesive powder and is fluidically connectable to a reservoir (16, 26, 36) comprising the liquid, wherein the plunger is configured to fluidically separate in a first position the reservoir and the mixing compartment and to fluidically connect in a second position the reservoir and the mixing compartment.

2. The bone adhesive applicator (10, 20, 30) according to claim 1, comprising a mixer (14, 24, 34) configured to mix the bone adhesive powder (2) and the liquid (1) in the mixing compartment (12, 22, 32), the mixer comprising a mixing head (141, 341) arranged in the mixing compartment and a connector (142, 342) connected to the mixing head and comprising a drive interface (1421, 3421), wherein the mixing head (141, 341) is preferably a brush, preferably comprising bristles made of a biodegradable material.

3. The bone adhesive applicator (10, 20, 30) according to claim 2, wherein the connector (1421, 3421) is guided through the outlet (111, 211, 311) such that the drive interface (1421, 3421) is arranged outside of the housing (11, 21, 31), wherein the drive interface is configured for connection with a dental handpiece.

4. The bone adhesive applicator (10, 20, 30) according to one of the preceding claims, wherein the housing (11, 21, 31) comprises a lateral inlet (112, 212, 314) configured for fluidic communication of the mixing compartment (12, 22, 32) with the reservoir (16, 26, 36), wherein the fluidic communication through the lateral inlet is interruptible by the plunger (13, 23, 33) in the first position of the plunger, wherein the lateral inlet preferably comprises an aperture (112, 212) in a lateral wall of the housing (11, 21), the aperture being fluidically closable by the plunger (13, 23) in the first position of the plunger, wherein the aperture (112, 212) preferably exhibits a diameter (d1) which is smaller than or equal to an axial diameter (D) of the plunger.

5. The bone adhesive applicator (10, 20) according to claim 4, comprising the reservoir (16, 26) which is arranged at an outer face of the housing (11, 21), wherein the reservoir preferably is a bag (16, 26) or a container, wherein the reservoir (16, 26) preferably comprises a reservoir outlet (161) which is flush with the aperture (112, 212) of the lateral inlet.

6. The bone adhesive applicator (10, 20) according to claim 4 or 5, wherein the mixing compartment (12, 22) and the plunger (13, 23) are configured to generate an intake pressure for liquid (1) at the lateral inlet (112, 212) by moving the plunger away from the outlet (111, 211).

7. The bone adhesive applicator (20) according to one of the claims 4 to 6, wherein a check valve (213) is arranged at the lateral inlet (212) or the reservoir outlet, wherein the check valve is configured to provide substantially only flow into the housing (21) or to provide a larger flow into the housing than out of the housing, wherein the check valve preferably comprises a flap (213) with an edge portion mounted at a side of the aperture (212) proximal to the plunger (23) in the second position.

8. The bone adhesive applicator (10, 20) according to one of the claims 4 to 7, wherein a cross-sectional area of the aperture (112, 212) of the lateral inlet is smaller than a cross-sectional area of the outlet (111, 211), preferably with a ratio of smaller than 1:5, particularly preferably smaller than 1:10.

9. The bone adhesive applicator (10, 20) according to one of the claims 4 to 8, wherein the lateral inlet (112, 212) comprises an interface configured for connection with a reservoir interface, wherein the interface preferably comprises a thread or one or more catches and/or recesses.

10. The bone adhesive applicator (30) according to claim 4, wherein the lateral inlet comprises a bulge (314) of a lateral wall of the housing (31) such that an inner diameter of the housing is increased at the bulge, wherein the bulge (314) preferably extends only around a part of the circumference of the housing (31), preferably around less than one sixteenth, one eighth or one quarter of the circumference of the housing, or preferably comprising two, three, four or more bulges which are evenly arranged around the circumference of the housing, wherein the bulges preferably each extend around less than one sixteenth, one eighth or one quarter of the circumference.

11. The bone adhesive applicator (30) according to claim 10, comprising an axially movable auxiliary plunger (37) arranged in series with the plunger (33) such that the plunger is arranged between the auxiliary plunger and the outlet (311), wherein the reservoir (36) is preferably formed between the auxiliary plunger (37) and the plunger (33).

12. The bone adhesive applicator (30) according to claim 11, wherein in the first position, the plunger (33) is at least partially arranged in a portion of the housing (31) adjacent to an axially distal end of the bulge (314) with respect to the outlet (311), wherein the auxiliary plunger (37) is preferably configured to displace the plunger (33) into the second position by moving towards the plunger.

13. The bone adhesive applicator (30) according to claim 12, wherein in the second position, the plunger (33) is arranged at the bulge (314) such that a fluid bypass is formed between the reservoir (36) and the mixing compartment (32), wherein the auxiliary plunger (37) is configured to transfer liquid (1) from the reservoir to the mixing compartment through the bypass by moving towards the plunger.

14. The bone adhesive applicator (30) according to one of the claims 11 to 13, wherein the auxiliary plunger (37) is movable towards the plunger (33) until contacting the plunger.

15. The bone adhesive applicator (10, 20, 30) according to one of the preceding claims, wherein the reservoir (16, 26, 36) has a smaller volume than the mixing compartment (12, 22, 32) when the plunger (13, 23, 33) is in the first position.

16. The bone adhesive applicator (10, 20, 30) according to one of the preceding claims, wherein the bone adhesive applicator is a dental bone adhesive applicator (10, 20, 30) for mixing a bone adhesive powder (2) with a liquid (1) to obtain a dental bone adhesive (3) and applying the dental bone adhesive.

17. A method for preparing a bone adhesive (3), comprising the steps of:
- providing a bone adhesive applicator (10, 20, 30) according to claim 2 or claim 2 and one of the preceding claims;
- supplying the liquid (1) from the reservoir (16, 26, 36) to the mixing compartment (12, 22, 32) by moving the plunger (13, 23, 33) from the first position to the second position;
- connecting a drive to the drive interface (1421, 3421) of the connector (142, 342), wherein the drive is preferably a drive of a dental handpiece;
- mixing the liquid with the bone adhesive powder (2) in the mixing compartment by actuating the mixer (14, 24, 34) using the drive.

## Patentansprüche

1. Ein Knochenkleber-Applikator (10, 20, 30) zum Mischen eines Knochenkleberpulvers (2) mit einer Flüssigkeit (1), um einen Knochenkleber (3) zu erhalten, und zum Auftragen des Knochenklebers, umfassend ein Gehäuse (11, 21, 31) mit einer Längsachse (L) und einem Auslass (111, 211, 311), eine im Gehäuse angeordnete und mit dem Auslass in Fluidverbindung stehende Mischkammer (12, 22, 32), einen axial beweglichen Kolben (13, 23, 33), der in dem Gehäuse angeordnet ist und die Mischkammer dem Auslass gegenüberliegend axial begrenzt, wobei die Mischkammer das Knochenkleberpulver enthält und fluidisch mit einem die Flüssigkeit enthaltenden Reservoir (16, 26, 36) verbindbar ist, wobei der Kolben so ausgebildet ist, dass er in einer ersten Position das Reservoir und die Mischkammer fluidisch voneinander trennt und in einer zweiten Position das Reservoir und die Mischkammer fluidisch miteinander verbindet.

2. Der Knochenkleber-Applikator (10, 20, 30) nach Anspruch 1, umfassend einen Mischer (14, 24, 34), der ausgebildet ist, das Knochenkleberpulver (2) und die Flüssigkeit (1) in der Mischkammer (12, 22, 32) zu mischen, wobei der Mischer einen im Mischraum angeordneten Mischkopf (141, 341) und ein mit dem Mischkopf verbundenes Verbindungsstück (142, 342) umfasst, das eine Antriebsschnittstelle (1421, 3421) aufweist, wobei der Mischkopf (141, 341) vorzugsweise eine Bürste ist, die vorzugsweise Borsten aus einem biologisch abbaubaren Material umfasst.

3. Der Knochenkleber-Applikator (10, 20, 30) nach Anspruch 2, wobei das Verbindungsstück (1421, 3421) so durch den Auslass (111, 211, 311) geführt ist, dass die Antriebsschnittstelle (1421, 3421) außerhalb des Gehäuses (11, 21, 31) angeordnet ist, wobei die Antriebsschnittstelle für den Anschluss an ein zahnärztliches Handstück ausgelegt ist.

4. Knochenkleber-Applikator (10, 20, 30) nach einem der vorstehenden Ansprüche, wobei das Gehäuse (11, 21, 31) einen seitlichen Einlass (112, 212, 314) aufweist, der zur fluidischen Verbindung der Mischkammer (12, 22, 32) mit dem Reservoir (16, 26, 36) ausgebildet ist, wobei die fluidische Verbindung durch den seitlichen Einlass durch den Kolben (13, 23, 33) in der ersten Position des Kolbens unterbrechbar ist, wobei der seitliche Einlass vorzugsweise eine Öffnung (112, 212) in einer Seitenwand des Gehäuses (11, 21) umfasst, wobei die Öffnung durch den Kolben (13, 23) in der ersten Position des Kolbens fluidisch verschließbar ist, wobei die Öffnung (112, 212) vorzugsweise einen Durchmesser (d1) aufweist, der kleiner oder gleich einem axialen Durchmesser (D) des Kolbens ist.

5. Der Knochenkleber-Applikator (10, 20) nach Anspruch 4, umfassend das Reservoir (16, 26), das an einer Außenfläche des Gehäuses (11, 21) angeordnet ist, wobei das Reservoir vorzugsweise ein Beutel (16, 26) oder ein Behälter ist, wobei das Reservoir (16, 26) vorzugsweise einen Reservoirauslass (161) aufweist, der bündig mit der Öffnung (112, 212) des seitlichen Einlasses ist.

6. Der Knochenkleber-Applikator (10, 20) nach Anspruch 4 oder 5, wobei die Mischkammer (12, 22) und der Kolben (13, 23) so ausgebildet sind, dass sie durch Wegbewegen des Kolbens vom Auslass (111, 211) einen Ansaugdruck für Flüssigkeit (1) am seitlichen Einlass (112, 212) erzeugen.

7. Der Knochenkleber-Applikator (20) nach einem der Ansprüche 4 bis 6, wobei am seitlichen Einlass (212) oder am Auslass des Reservoirs ein Rückschlagventil (213) angeordnet ist, wobei das Rückschlagventil so ausgelegt ist, dass es im Wesentlichen nur einen Durchfluss in das Gehäuse (21) bereitstellt oder einen größeren Durchfluss in das Gehäuse als aus dem Gehäuse heraus bereitstellt, wobei das Rückschlagventil vorzugsweise eine Lasche (213) mit einem Randabschnitt aufweist, der an einer Seite der Öffnung (212) proximal zum Kolben (23) in der zweiten Position angebracht ist.

8. Der Knochenkleber-Applikator (10, 20) nach einem der Ansprüche 4 bis 7, wobei die Querschnittsfläche der Öffnung (112, 212) des seitlichen Einlasses kleiner ist als die Querschnittsfläche des Auslasses (111, 211), vorzugsweise mit einem Verhältnis von weniger als 1:5, besonders bevorzugt von weniger als 1:10.

9. Der Knochenkleber-Applikator (10, 20) nach einem der Ansprüche 4 bis 8, wobei der seitliche Einlass (112, 212) eine Schnittstelle aufweist, die zur Verbindung mit einer Reservoir-Schnittstelle ausgelegt ist, wobei die Schnittstelle vorzugsweise ein Gewinde oder eine oder mehrere Rastvorrichtungen und/oder Aussparungen umfasst.

10. Der Knochenkleber-Applikator (30) nach Anspruch 4, wobei der seitliche Einlass eine Ausbuchtung (314) einer Seitenwand des Gehäuses (31) umfasst, so dass der Innendurchmesser des Gehäuses an der Ausbuchtung vergrößert ist, wobei sich die Ausbuchtung (314) vorzugsweise nur um einen Teil des Umfangs des Gehäuses (31) erstreckt, vorzugsweise um weniger als ein Sechzehntel, ein Achtel oder ein Viertel des Umfangs des Gehäuses, oder vorzugsweise zwei, drei, vier oder mehr Ausbuchtungen umfasst, die gleichmäßig um den Umfang des Gehäuses angeordnet sind, wobei sich die Ausbuchtungen vorzugsweise jeweils um weniger als ein Sechzehntel, ein Achtel oder ein Viertel des Umfangs erstrecken.

11. Der Knochenkleber-Applikator (30) nach Anspruch 10, umfassend einen axial beweglichen Hilfskolben (37), der in Reihe mit dem Kolben (33) angeordnet ist, sodass sich der Kolben zwischen dem Hilfskolben und dem Auslass (311) befindet, wobei das Reservoir (36) vorzugsweise zwischen dem Hilfskolben (37) und dem Kolben (33) ausgebildet ist.

12. Der Knochenkleber-Applikator (30) nach Anspruch 11, wobei sich der Kolben (33) in der ersten Position zumindest teilweise in einem zu einem in Bezug auf den Auslass (311) axial distalen Ende der Ausbuchtung (314) benachbarten Abschnitt des Gehäuses (31) befindet, wobei der Hilfskolben (37) vorzugsweise so ausgebildet ist, dass er den Kolben (33) durch eine Bewegung in Richtung des Kolbens in die zweite Position verschiebt.

13. Der Knochenkleber-Applikator (30) nach Anspruch 12, wobei sich der Kolben (33) in der zweiten Position an der Ausbuchtung (314) derart befindet, dass zwischen dem Reservoir (36) und der Mischkammer (32) ein Fluid-Bypass gebildet wird, wobei der Hilfskolben (37) so ausgebildet ist, dass er durch eine Bewegung in Richtung des Kolbens Flüssigkeit (1) aus dem Reservoir in die Mischkammer durch den Bypass befördert.

14. Der Knochenkleber-Applikator (30) nach einem der Ansprüche 11 bis 13, wobei der Hilfskolben (37) in Richtung des Kolbens (33) bewegbar ist, bis er den Kolben berührt.

15. Der Knochenkleber-Applikator (10, 20, 30) nach einem der vorstehenden Ansprüche, wobei das Resevoir (16, 26, 36) ein kleineres Volumen als die Mischkammer (12, 22, 32) aufweist, wenn sich der Kolben (13, 23, 33) in der ersten Position befindet.

16. Der Knochenkleber-Applikator (10, 20, 30) nach einem der vorstehenden Ansprüche, wobei der Knochenkleber-Applikator ein Dental-Knochenkleber-Applikator (10, 20, 30) zum Mischen eines Knochenkleberpulvers (2) mit einer Flüssigkeit (1) zur Herstellung eines Dental-Knochenklebers (3) und zum Auftragen des Dental-Knochenklebers ist.

17. Verfahren zur Herstellung eines Knochenklebers (3), umfassend die folgenden Schritte:
- Bereitstellen eines Knochenkleber-Applikators (10, 20, 30) nach Anspruch 2 oder Anspruch 2 und einem der vorstehenden Ansprüche;
- Zuführen der Flüssigkeit (1) aus dem Reservoir (16, 26, 36) in die Mischkammer (12, 22, 32) durch Bewegen des Kolbens (13, 23, 33) von der ersten Position in die zweite Position;
- Anschließen eines Antriebs an die Antriebsschnittstelle (1421, 3421) des Verbindungsstücks (142, 342), wobei der Antrieb vorzugsweise ein Antrieb eines zahnärztlichen Handstücks ist;
- Mischen der Flüssigkeit mit dem Knochenkleberpulver (2) in der Mischkammer durch Betätigen des Mischers (14, 24, 34) mittels des Antriebs.

## Revendications

1. Applicateur d'adhésif osseux (10, 20, 30) destiné à mélanger une poudre d'adhésif osseux (2) avec un liquide (1) afin d'obtenir un adhésif osseux (3) et à appliquer ledit adhésif osseux, comprenant un boîtier (11, 21, 31) présentant un axe longitudinal (L) et une sortie (111, 211, 311), un compartiment de mélange (12, 22, 32) disposé dans le boîtier et en communication fluidique avec la sortie, un piston mobile axialement (13, 23, 33) disposé dans le boîtier et délimitant axialement le compartiment de mélange à l'opposé de la sortie, dans lequel le compartiment de mélange contient la poudre d'adhésif osseux et peut être relié de manière fluidique à un réservoir (16, 26, 36) contenant le liquide, dans lequel le piston est configuré pour séparer de manière fluidique, dans une première position, le réservoir et le compartiment de mélange, et pour relier de manière fluidique, dans une deuxième position, le réservoir et le compartiment de mélange.

2. L'applicateur d'adhésif osseux (10, 20, 30) selon la revendication 1, comprenant un mélangeur (14, 24, 34) configuré pour mélanger la poudre d'adhésif osseux (2) et le liquide (1) dans le compartiment de mélange (12, 22, 32), le mélangeur comprenant une tête de mélange (141, 341) disposée dans le compartiment de mélange et un connecteur (142, 342) relié à la tête de mélange et comprenant une interface d'entraînement (1421, 3421), dans lequel la tête de mélange (141, 341) est de préférence une brosse, comprenant de préférence des poils en un matériau biodégradable.

3. L'applicateur d'adhésif osseux (10, 20, 30) selon la revendication 2, dans lequel le connecteur (1421, 3421) est guidé à travers la sortie (111, 211, 311) de telle sorte que l'interface d'entraînement (1421, 3421) soit disposée à l'extérieur du boîtier (11, 21, 31), l'interface d'entraînement étant configurée pour être raccordée à une pièce à main dentaire.

4. L'applicateur d'adhésif osseux (10, 20, 30) selon l'une des revendications précédentes, dans lequel le boîtier (11, 21, 31) comprend une entrée latérale (112, 212, 314) configurée pour assurer une communication fluidique entre le compartiment de mélange (12, 22, 32) et le réservoir (16, 26, 36), dans lequel la communication fluidique à travers l'entrée latérale peut être interrompue par le piston (13, 23, 33) dans la première position de celui-ci, dans lequel l'entrée latérale comprend de préférence une ouverture (112, 212) dans une paroi latérale du boîtier (11, 21), l'ouverture pouvant être obturée par le piston (13, 23) de manière fluidique dans la première position de celui-ci, dans lequel l'ouverture (112, 212) présente de préférence un diamètre (d1) inférieur ou égal au diamètre axial (D) du piston.

5. L'applicateur d'adhésif osseux (10, 20) selon la revendication 4, comprenant le réservoir (16, 26) qui est disposé sur une face extérieure du boîtier (11, 21), dans lequel le réservoir est de préférence un sac (16, 26) ou un récipient, dans lequel le réservoir (16, 26) comprend de préférence une sortie de réservoir (161) affleurant à l'ouverture (112, 212) de l'entrée latérale.

6. L'applicateur d'adhésif osseux (10, 20) selon la revendication 4 ou 5, dans lequel le compartiment de mélange (12, 22) et le piston (13, 23) sont configurés pour générer une pression d'aspiration du liquide (1) au niveau de l'entrée latérale (112, 212) en éloignant le piston de la sortie (111, 211).

7. L'applicateur d'adhésif osseux (20) selon l'une des revendications 4 à 6, dans lequel un clapet anti-retour (213) est disposé au niveau de l'entrée latérale (212) ou de la sortie du réservoir, ledit clapet anti-retour étant configuré pour permettre essentiellement uniquement un écoulement vers le boîtier (21) ou pour permettre un écoulement plus important vers le boîtier qu'à partir de celui-ci, ledit clapet anti-retour comprenant de préférence un volet (213) dont une partie de bord est montée sur un côté de l'ouverture (212) proximal au piston (23) dans la deuxième position.

8. L'applicateur d'adhésif osseux (10, 20) selon l'une des revendications 4 à 7, dans lequel la section transversale de l'ouverture (112, 212) de l'entrée latérale est inférieure à celle de la sortie (111, 211), de préférence avec un rapport inférieur à 1:5, de manière particulièrement préférée inférieur à 1:10.

9. L'applicateur d'adhésif osseux (10, 20) selon l'une des revendications 4 à 8, dans lequel l'entrée latérale (112, 212) comprend une interface configurée pour être raccordée à une interface de réservoir, ladite interface comprenant de préférence un filetage ou un ou plusieurs ergots et/ou évidements.

10. L'applicateur d'adhésif osseux (30) selon la revendication 4, dans lequel l'entrée latérale comprend un renflement (314) d'une paroi latérale du boîtier (31) de telle sorte que le diamètre intérieur du boîtier soit accru au niveau du renflement, le renflement (314) s'étendant de préférence uniquement sur une partie de la circonférence du boîtier (31), de préférence sur moins d'un seizième, d'un huitième ou d'un quart de la circonférence du boîtier, ou comprenant de préférence deux, trois, quatre renflements ou plus qui sont répartis uniformément sur la circonférence du boîtier, les renflements s'étendant de préférence chacun sur moins d'un seizième, d'un huitième ou d'un quart de la circonférence.

11. L'applicateur d'adhésif osseux (30) selon la revendication 10, comprenant un piston auxiliaire (37) mobile axialement, disposé en série avec le piston (33) de telle sorte que le piston soit situé entre le piston auxiliaire et la sortie (311), le réservoir (36) étant de préférence formé entre le piston auxiliaire (37) et le piston (33).

12. L'applicateur d'adhésif osseux (30) selon la revendication 11, dans lequel, dans la première position, le piston (33) est disposé au moins partiellement dans une partie du boîtier (31) adjacente à une extrémité axialement distale du renflement (314) par rapport à la sortie (311), le piston auxiliaire (37) étant de préférence configuré pour déplacer le piston (33) vers la deuxième position en se déplaçant vers le piston.

13. L'applicateur d'adhésif osseux (30) selon la revendication 12, dans lequel, dans la deuxième position, le piston (33) est disposé au niveau du renflement (314) de telle sorte qu'une dérivation de fluide soit formée entre le réservoir (36) et le compartiment de mélange (32), le piston auxiliaire (37) étant agencé pour transférer le liquide (1) du réservoir vers le compartiment de mélange à travers cette dérivation en se déplaçant vers le piston.

14. L'applicateur d'adhésif osseux (30) selon l'une des revendications 11 à 13, dans lequel le piston auxiliaire (37) est mobile vers le piston (33) jusqu'à entrer en contact avec celui-ci.

15. L'applicateur d'adhésif osseux (10, 20, 30) selon l'une des revendications précédentes, dans lequel le réservoir (16, 26, 36) présente un volume inférieur à celui du compartiment de mélange (12, 22, 32), lorsque le piston (13, 23, 33) se trouve dans la première position.

16. L'applicateur d'adhésif osseux (10, 20, 30) selon l'une des revendications précédentes, dans lequel l'applicateur d'adhésif osseux est un applicateur d'adhésif osseux dentaire (10, 20, 30) destiné à mélanger une poudre d'adhésif osseux (2) avec un liquide (1) pour obtenir un adhésif osseux dentaire (3) et à appliquer ledit adhésif osseux dentaire.

17. Procédé de préparation d'un adhésif osseux (3), comprenant les étapes:
- fournir un applicateur d'adhésif osseux (10, 20, 30) selon la revendication 2 ou la revendication 2 et l'une des revendications précédentes;
- l'alimentation en liquide (1) depuis le réservoir (16, 26, 36) vers le compartiment de mélange (12, 22, 32) en déplaçant le piston (13, 23, 33) de la première position à la deuxième position;
- raccorder un entraînement à l'interface d'entraînement (1421, 3421) du connecteur (142, 342), l'entraînement étant de préférence celui d'une pièce à main dentaire;
- mélanger le liquide avec la poudre d'adhésif osseux (2) dans le compartiment de mélange en actionnant le mélangeur (14, 24, 34) à l'aide de l'entraînement.
